# EUROPEAN PATENT APPLICATION

(11) **EP 1 064 882 A1**
(43) Date of publication of application: **03.01.2001**
(21) Application number: 00901144.6
(22) Date of filing: 20.01.2000
(51) Int. Cl.: A61B 17/00

(54) **DEVICE AND METHOD FOR GUIDED PERCUTANEOUS APPLICATION OF A RETRACTILE STAPLE OR A HEMOSTATIC MATERIAL USING A NOVEL INSERTION SYSTEM THAT OPENS UPON WITHDRAWAL AND ENABLES OPERATING CARDIOLOGISTS AND ENDOSCOPIC SURGEONS TO EASILY, RAPIDLY AND RELIABLY ACHIEVE HEMOSTASIS IN THE PUNCTURE SITE**

(30) Priority: 21.01.1999 ES 9900118
(71) Applicant: Biomed S.A., 28760 Tres Cantos (ES)
(72) Inventor: MORENO FERNANDEZ DE BETONO, Ricardo, E-28760 Tres Cantos- Madrid (ES)
(74) Representative: Urizar Anasagasti, José Antonio
(86) International application number: ES0000022
(87) International publication number: WO0042917

(57) **Abstract**

The invention relates to a device and to a method for guided percutaneous application of a retractile staple or a haemostatic material using a novel insertion system that opens upon withdrawal and enables operating cardiologists and endoscopic surgeons to easily, rapidly and reliably achieve haemostasis in the puncture site, due to the fact that said system is a guided system. Said haemostatic device is called |REPERGUST| (from english language: REtractile, PERcutaneous, GUided, STaple) and provides the following innovative advantages: 1) precision in percutaneous placement due to the fact that the staple is guided towards the orifice that is to be closed by said guide exiting from said orifice, which guide has enabled the realization of various diagnostic and therapeutic operations. 2) immediate mechanical closure does not require waiting time for the coagulum to form and consolidate thereby enabling the patient to resume his or her normal activity right away; 3) the retractile nature of the staple, i.e. the retraction that takes place while the orifice is closing, is an important safety element since it avoids having to incorporate the adjacent structures (e.g. the wall opposite to the vessel) 4) access by the device carrying the staple to the orifice that is to be closed is facilitated by a novel introducing device that opens when it is withdrawn: 5) this easy and rapid closing system can be very useful for closing the orifices remaining on the wall of the vessel after conducting various percutaneous operations by means of guides such as angiography, angioplasty and other surgical procedures; 6) said system can also be very useful in laparoscopic surgery when it is required to make a stitch or apply a staple to close orifices, incisions, sutural defects or lacerations in cavities, vessels, tracts or structures.

## Description

### OBJECT FIELD OF THE INVENTION:

The main object of the present invention is the percutaneous closure of the vessel in the puncture site after using the SELDINGER technique, i.e the percutaneous insertion of a guide to perform an peripheric or coronary angiography, percutaneous transluminal angioplasty, coronary stent placement or other surgical procedures.

The increasing of these percutaneous techniques, often with catheters or more and more thicker devices makes it necessary to dispose of a simple, quick and secure device to provide stable and immediate haemostasis at the puncture site, even on patients with anticoagulant therapy.

Furthermore, the more and more frequent usage of laparoscopic and minimally invasive surgery techniques produces many occasions in which it would be very useful to have a device that provides for accurate percutaneous application of a staple that can close orifices, incisions, suture defects or lacerations in cavities, vessels, conducts or structures of the human body.-

### BACKGROUND OF THE INVENTION

Along the last years, the use of the SELDINGER technique has extraordinary increased in interventionist cardiology, for diagnostic and as well as for therapeutic purposes.

There exists a potential of more than 7 MILLION interventions of this type that are performed annually in the whole world.

As an inconvenience, the SELDINGER technique shows up the closure of the arterial puncture right after the procedure. In the past, the current standard of care was to apply manual pressure for approximately 30 minutes, being advisable a further bed rest of 8 to 12 hours while waiting for the coagulum to be formed.

This simple method is not applicable when the use of more and more thicker catheters or intravascular devices becomes necessary. Its use on anticoagulated patients becomes also nonviable.

It is then necessary to provide a device that enables interventional cardiologist to achieve post-procedure haemostasis at the puncture site in a quicker and more effective way than with conventional methods.

Nowadays there exist several systems for vascular sealing. The main characteristics of the most important ones are listed hereafter for the purpose of establishing a comparison with the present invention:

### 1) Manual pressure and bed rest.

It is a very useful and simple procedure but only when dealing with very thin catheters and with patients not anticoagulated.

### 2) Mechanical external pressure and bed rest

There are many procedures to maintain mechanical pressure at the puncture site: From the primitive sand bags to the |Apron Like| of KURTH, (US Patent nr. 4.829.994), that includes a stiff band to compress the upper part of the thigh. This avoids the nurse's manual action, but the application of the device is cumbersome, and there is a risk of ischemic problems.

Another system of this kind is the one described by SINOFSKY, (US Patent nº 4.929.246), that applies external mechanical compression plus thermal laser effect on the puncture site.

### 3) Collagen or fibrine plugs applied at the puncture site

In the last years, there have been reported several systems of vascular sealing of the puncture site employing collagen or fibrine plugs. The proceedings that leave part of the occlusive system inside the vessel have not been widely used because of the potential risk of intravascular thrombosis : KENSEY (US Pats. 4.744.364 and 4.890.612). U.T. AKER et al. with his method denominated HPCD (From : Haemostatic, Puncture, Closure, Device).

Among the methods that employ collagen plugs, but only in the outside of the puncture site, the most popular is the |Vasoseal|, with two collagen plugs, placed one after the other. The application must be preceded by some rather complex actions, directed to facilitate the precise placement of the plugs.

### 4. Direct closure of the puncture site using minimally invasive surgery.

One procedure of this type is the Techstar. |Percutaneous Vascular Device| of PERCLOSE (Menlo Park, Cal.). A small subcutaneous dilatator is used to enlarge the cutaneous perforation and the subcutaneous tunnel and facilitate the introduction of the suture instrument with two needles and a polyester suture.

### DESCRIPTION OF THE INVENTION

In order to prevent said inconveniences, there has been developed a procedure and a device consisting of using the conduction of the guide and an inserting instrument that allow for a staple or haemostatic material to precisely reach the desired point for the percutaneous closure of orifices, incisions or lacerations in vessels, conducts or structures, wherein said device and its application procedure form the object of the present invention.

The device of the present invention is directed to place a retractile staple or haemostatic material in the site of arterial puncture, thereby making good use of the guide left after the mentioned Seldinger technique. Said apparatus is formed of two parts or units, one of them sliding inside the other. The external unit consists of a tube formed out of two sections of different diameters; The section with the larger diameter is provided with wings that project themselves towards the outside and are designed as a support for the fingers and have their inside prepared to house a compression spring: The section with the smaller diameter has in its free end two bumpers or pivots that will be used for the inflection, closure and retraction oft the retractile staple when the instrument is activated, and shows a longitudinal slot directed to allow the exit of the mentioned guide. The inner unit consists of a piston with a suitable diameter for being housed and slide with accuracy inside the external unit Its proximal end is finished up with a projection, preferably conic-shaped, like a knob designed to be pressed by the thumb. The distal end shows a longitudinal internal channel for the guide pass through, ending up in a spindle-shaped offset with several lateral projections for holding the retractile staple, and bears in its central area a protuberance acting as a bumper to the mentioned compression spring.

In the coupling of the depicted inner and outer units, the knob actuates jointly with the wings to achieve the longitudinal movement of the internal element with respect to the external unit, the original position being recovered due to the compression spring action. Likewise, the existing slot in the external unit corresponds to the inner channel exit of the internal unit in order to allow withdrawing of the guide without any obstacles.

In the resting position, the spring remains in the position of maximal expansion, hereof resulting that the apparatus shows its maximal length; By activating the apparatus, acting with the fingers of one hand, the spring is compressed and the length of the device is progressively shortened to a minimal value or of maximal compression; If actuation stops the apparatus turns back to its initial position due to the spring effect.

Another object of the present invention is a retractile staple formed of a plate, preferably rectangular, with a central orifice for the guide passing through, and several tips, preferably four, which will penetrate into the tissue in order to perform the closure. Said retractile staple shows a perimetric prominence suitable shaped for being engaged into the correspondent notch located in the forwarding front end of said internal unit of the apparatus. Said staple is formed in such a way that when the bumpers allocated in the forwarding end of said unit of the apparatus do contact, after penetrating into the tissue to be sutured it folds back achieving the closure of the orifice, while in the same time retracting.

Another object of the present invention is the procedure for the application of said retractile staple or haemostatic material by means of said apparatus, said procedure consisting of the placing of said retractile staple into the notch allocated in the forwarding end of said apparatus, the subcutaneous inserting of said staple using the guide used in the surgical intervention previously performed, the penetration of the tips of said staple into the tissue to be sutured and the fold back of it by means of the simultaneous action of the internal and external units of said apparatus as the internal unit moves inside the external unit in the way previously described. If a haemostatic material replaces the staple, the said material remains located with precision.

Another object of the present invention is an ***introduceing device,*** which facilitates the access to the closure instrument initially through the skin and subcutaneous tissue just up to its positioning on the external wall of the vessel, in the puncture area. The introducing device, preferably made of rigid plastic material, consists of a tube whose interior section adapts to the section with the minor diameter of the external tube of the closure instrument, and is of such size that the movement of the introduction system on said tube can be performed smoothly and without any kind of friction. Longitudinally, there exists in the tube a slot that when overlapped lines up to the slot of the closure instrument, and through which the guide is released. One of the ends of the introducing device ends up as a tip, preferably with an obtuse shape, and with a central orifice for the threading of the guide and enough diameter as to allow its getting through. The obtuse tip shows slots or channels, in an optimal number of 2 or 4, with a reduced thickness of the wall in relation to the thickness of the rest of the tube, in order to achieve that in moving back the introducing device the forwarding end of the closure instrument tears the mentioned slots or channels permitting their back motion, remaining released and positioned over the arterial puncture site. The obtuse shape of the tip of the introducing device intends to favour the non-traumatic and precise inserting of the instrument. The other end of the introducing device shows wings projected towards the outside and is designed as a support for the fingers, whose relative orientation is 90º - shifted in relation to the supporting wings of the closure instrument.

This procedure shows important advantages in relation to known techniques. as follows:
a) The staple or haemostatic material is guided up to the orifice to be closed, caused by punction, by means of a guidewire that passes through said orifice, thereby being its percutaneous location very precise.
b) The procedure is applied immediately after the intravascular surgical intervention before withdrawing the guide, and as it is question of a mechanical closure or with a plug of haemostatic material, an immediate haemostasis is achieved without having to wait for the clot to form, thereby the patient being able to resume his or her normal activity in a short period.
c) The retraction motion of the staple while closing, increases the safety of the procedure, because it prevents damage of the closest structures like i.e. the opposite walls of the vessel.

This way, with the assembly of apparatus and retractile staple or haemostatic material and by means of the disclosed application procedure, it is achieved to solve the suture problem of orifices incisions or lacerations caused by surgical subcutanean interventions performed with the Seldinger or any other laparoscopic technique.

### DETAILED DESCRIPTION OF THE INVENTION

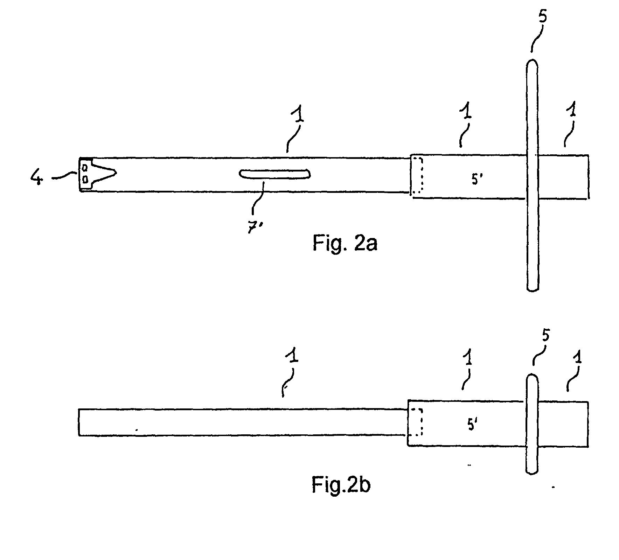

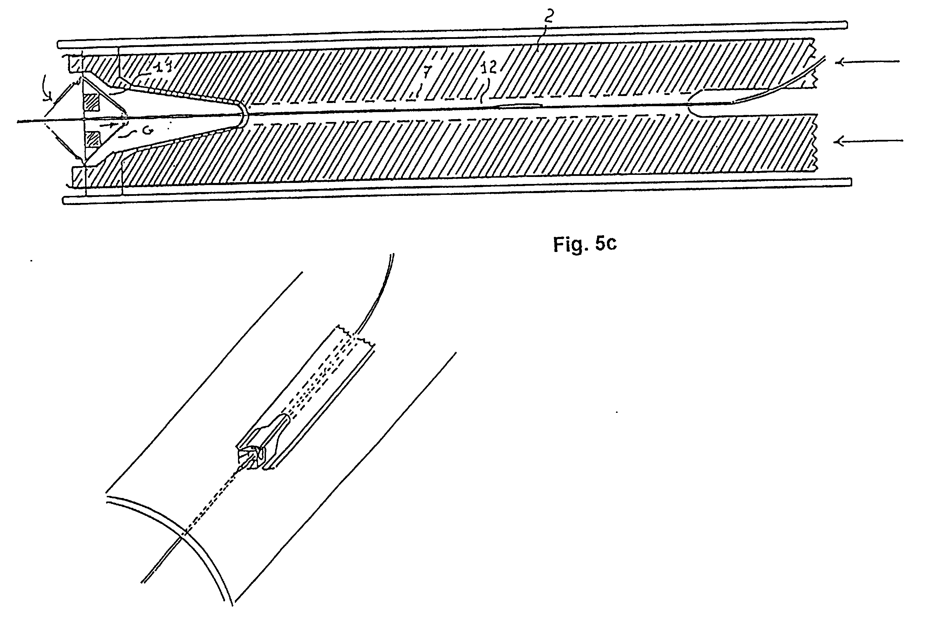

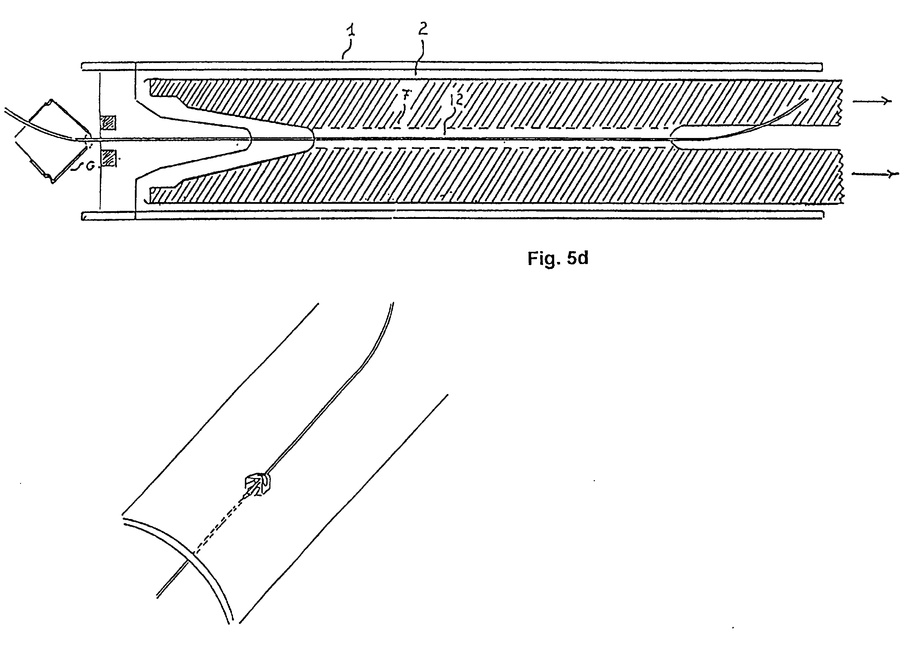

For a better understanding of the invention, reference is made to attached figures 1 to 14.
FIGURE 1. The complete device is shown in figure (1a) as a frontal elevational view and in (1b) in a lateral one.
   The device consists of two main parts: an external (1) and an internal (2) that slides inside the external. By transparency a spring (3) can be seen. This spring helps the internal part to recover its initial position, after the staple has been applied.
FIGURE 2. Shows the external part of the device, in frontal view, (2a) and in lateral view (2b).
   On the left side of figure (2a), two pivots (4) are shown, which cause the inflection, closing and retraction of the staple when the instrument is activated.
   On the other end, there are two small wings (5) to support the fingers when driving the device. The slot (7) permits the exit of the guide. A wider segment (5') houses the spring (Fig. 1ª).

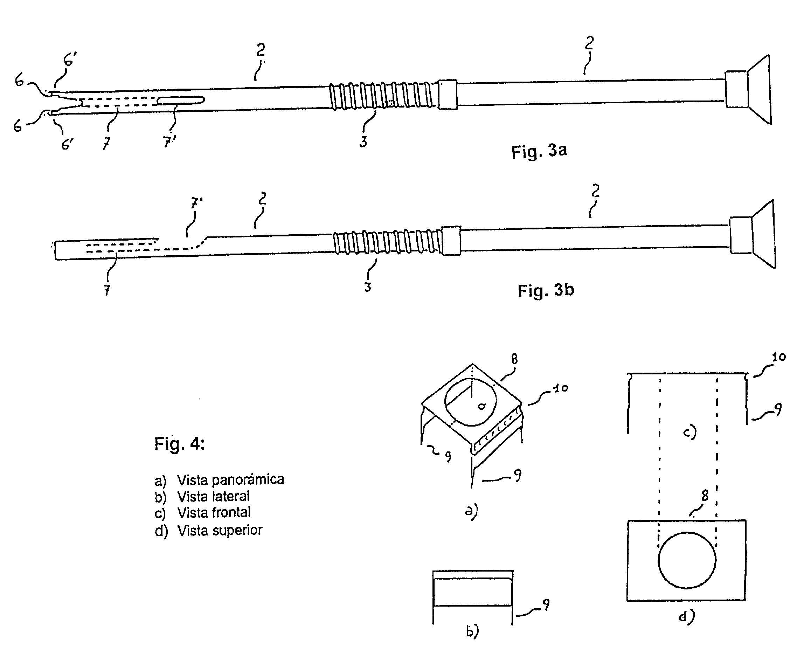

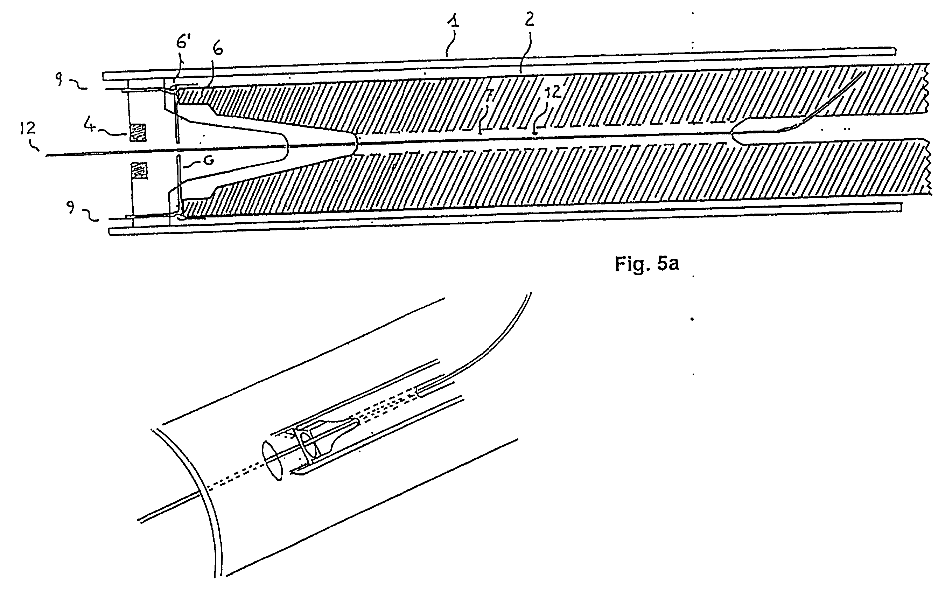

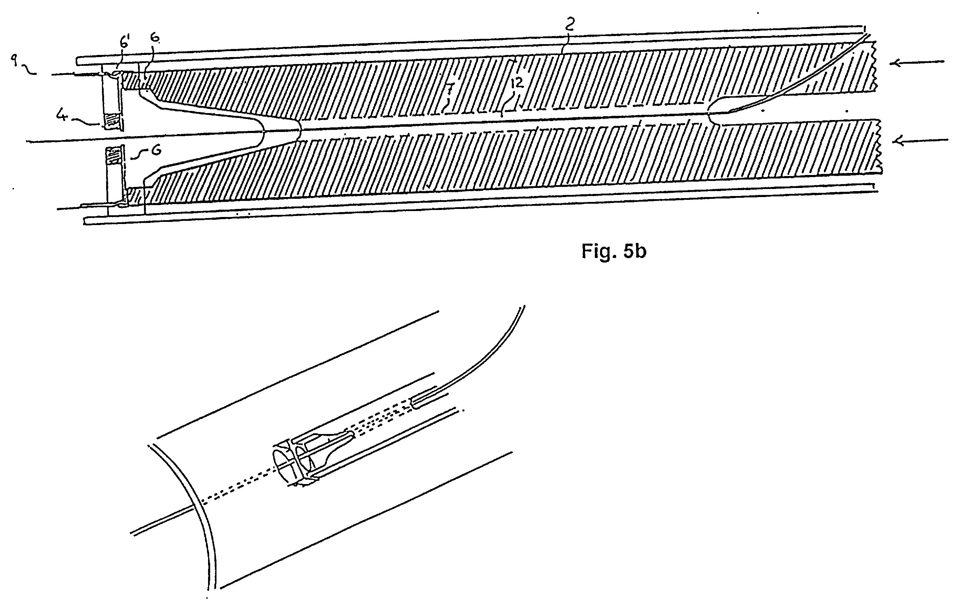

FIGURE 3. Shows the internal part of the device (3ª) in a frontal view and (3b) in lateral view respectively. On the left side of the figure, two tips (6) are shown, with two small hooks (6') to hold the staple and to cause, against the pivots, the inflection and closing of the staple.
FIGURE 4. Shows the shape of the staple in four positions: (a) is a perspective view showing the big central orifice that permits to thread the guide. The weakest part of the staple at (8), facilitates its inflection, retraction and closing at the desired point.
   The four sharp ends (9), as well as some small needles, are provided to hold the rim of the orifice to be closed, before the inflection of the staple. Said ends (9) are very short (0,4 mm). This shortness and the fact that the staple recoils while closing makes nearly impossible to damage the opposite wall when closing a puncture site in a vessel. (See the recoil movement in Fig. 5c).
   The small groove (10) on both sides of the staple, permits its temporal coupling with the tips (6) of the internal part.
   This type of coupling is not essential and can be deleted.
FIGURE 5. The figure represents the distal part of the device, enlarged tenfold, to show, in detail, the system that activates the staple.
FIGURE 5a. The device is at rest. The staple (G) is coupled to the tips (6) of the internal part. Between the pivots (4) there is a space for the guide (12). The tunnel (7) can also be seen.
   The sharp ends of the staple (9) are, at this moment, protected by the external part of the instrument (1) to facilitate its introduction through the skin and subcutaneous tissue.
FIGURE 5b. Shows the first phase of the device activation: The internal part (2) with the staple, has been moved as far as to contact the pivots (4). At this moment, the sharp ends of the staple are prominent and stuck to the rim of the orifice to be closed.
FIGURE 5c. Second phase of the device activation: It is the moment of INFLECTION and RETRACTION of the staple. When pushing the internal part (2), the staple is forced against the pivots sharp corners, that acting as fulcrums, cause the closure and recoil of the staple.
FIGURE 5d. The application is finished. The figure shows that both the staple and the puncture site are closed.
   The staple is cleared from the applicator, but still threaded on the guidewire, which is removed afterwards.

### THE INTRODUCER SYSTEM THAT OPENS WHEN PULLED BACK

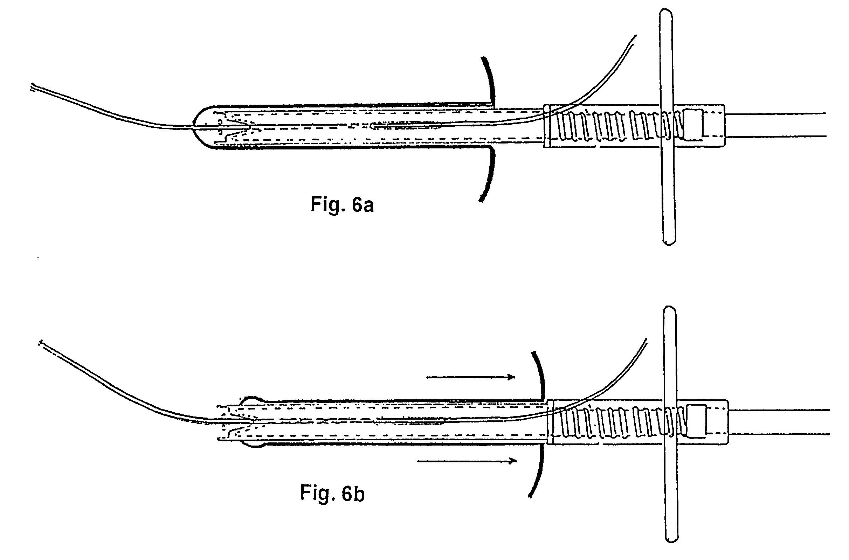

FIGURE 6. The initial and final position of introducing device system is shown.
FIGURE 6a. The introducer system is closed and threaded in the guidewire ready to be introduced through the skin and the subcutaneous tissue.
FIGURE 6b. The introducing device has been pulled back and the distal end is open permitting the advance of the staple bearing device.

### THE TOTALLY PLASTIC AND DISPOSABLE MODEL

FIGURE 7

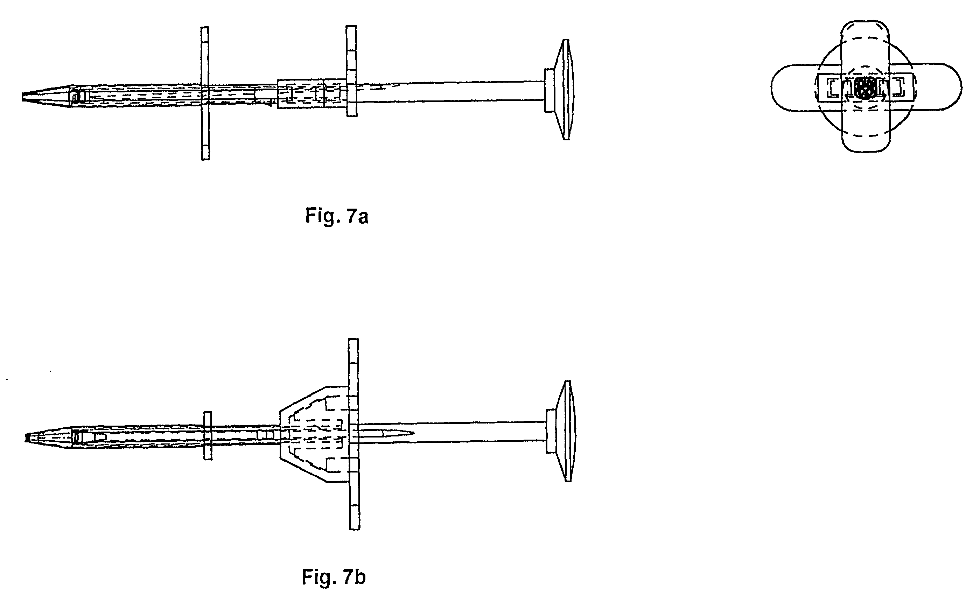

FIGURE 7a. shows the complete device, the plastic model in a side view.
FIGURE 7b. shows the complete device, the plastic model in a front view.
FIGURE 8

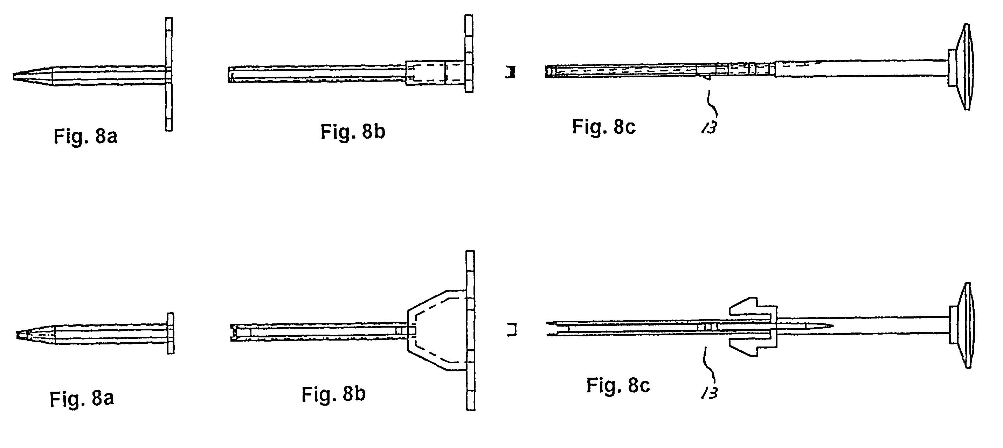

FIGURE 8a. shows the introducing device.
FIGURE 8b. shows the external unit
FIGURE 8c. shows the internal unit with the blocking system (13)

### A PREFERRED EMBODIMENT OF THE INVENTION

A preferred embodiment of the invention is disclosed, in which the model is completely made of plastic materials on order to convert it in a disposable one way device. In this embodiment, the closure instrument is loaded with one sole retractile staple incorporated at the time of its production or mounting.

The design is optimized by diminishing the number of components of the closure instrument down to 2 units or parts, plus the retractile staple and the mobile introducing device system. The ***internal unit*** shows ends of similar shape to those described in the previous embodiment of the invention, and with the same function: the holding of the staple and a knob for supporting and pressing by the thumb in order to activate the device. It is as well provided with a longitudinal slot for the exit of the guide. In the middle zone, the effect performed by the compression spring in the above-described embodiment is replaced by a system of locking pins. The external unit shows in its forwarding end the same configuration, and the opposite end consists of a box that externally contains the wings for supporting the fingers, and is internally a cavity with oblique lateral walls where the locking pins of the internal unit are coupled to. The output slot of the guide has been eliminated. giving an output to it through the posterior part of the box.

The relative longitudinal movement of the internal unit with respect to the external one is similarly achieved by pressure or activation on the knob of the first one, but in this embodiment, the longitudinal movement is limited by the allowed course for the locking pins inside the external unit box and the minimal flexion the plastic allows to them. Recuperation of the initial position in achieved by releasing the knob from pressure and through the tendency of the locking pins to regain their initial position of rest, free of tension.

The preferred embodiment of the invention incorporates a ***blocking system*** that avoids involuntary activation of the staple before the introducing device has been withdrawn. This blocking system consists of a |flange| allocated in the inside unit, in a foregoing position to the locking pins (from the side of the forwarding end) that is prominent through a window performed in the external unit when both units are coupled and so producing the blocking of them. When the introducing device system is moved backwards in order to release the housing zone of the staple, it pushes the flange to the inside, and when this one releases the window, it causes the unblocking and the relative movement of the external and internal units is facilitated.

In this preferred embodiment, the retractile staple and the mobile introducing device system show the same configuration as in the foregoing description of the invention. This preferred embodiment is also valid when haemostatic material is used.

### HOW TO USE THE DEVICE

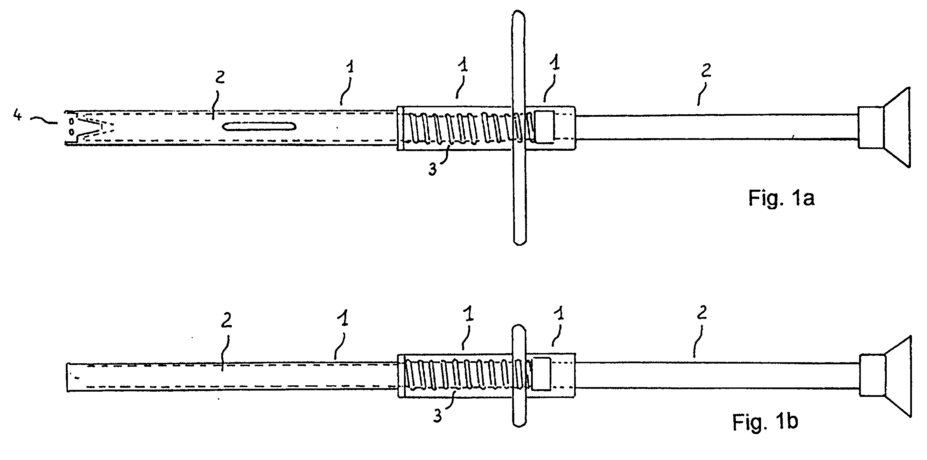

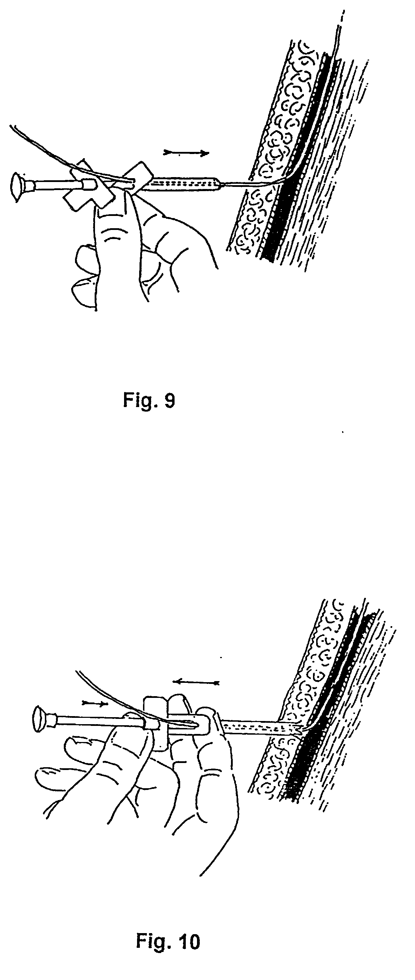

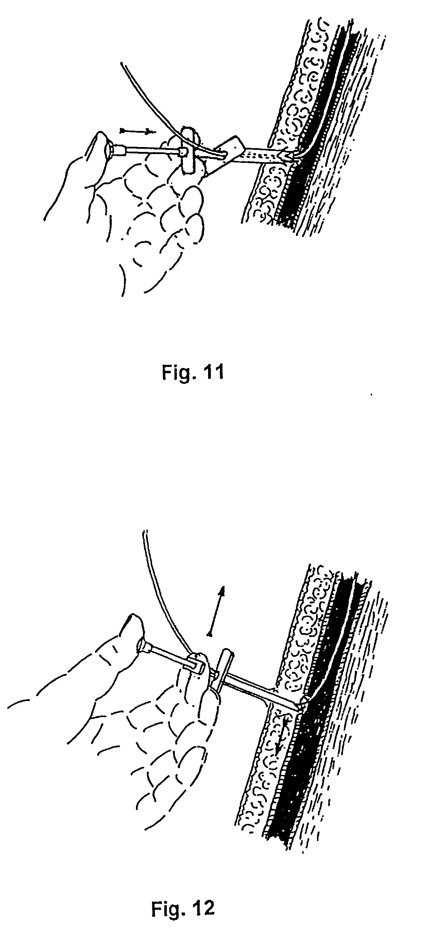

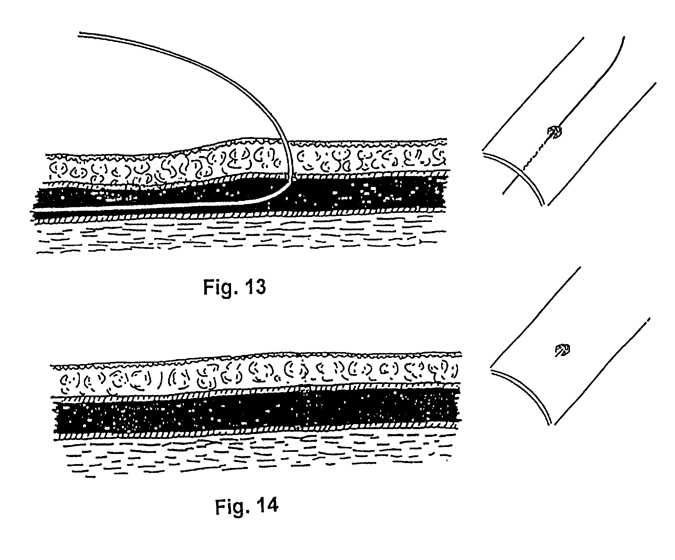

After a percutaneous intervention: angiography, transluminal angioplasty, coronary stent placement or other procedures, the dilatator or introducing device is taken out leaving the guide allocated in its place and the artery is pressed through the skin, near to the puncture site.
1. First, thread the introducing device into the guide and immediately after that, thread the instrument by passing the guide through the pivots, the orifice of the staple, the tunnel of the inner unit and the output slot (i.e fig. 1 and ff.) Couple the introducing device system to the closure instrument.
2. Lead the system along the guide and pierce the skin and subcutaneous tissue, until reaching the artery's surface in the puncture site. (fig. 9)
3. Move the introducing device back by pressing backwards on the supporting wings of it. (fig. 10).
4. Keeping the device against the artery with moderate pressure, push the inner unit by digital compression between the knob of this unit and the supporting wings of the external unit, as shown in fig. 11. The staple closes and retracts, closing the arterial puncture site. (figs. 5 a, b, c, d).
5. Before extracting the device (fig. 12) it is convenient to perform a light oscillating forward movement, what provokes a minimal displacement backwards of the distal end (close to the artery), in an exact manner, to insure that the staple already allocated in its place, does not move as the instrument is withdrawn.
6. The device (figs. 13 and 14) is withdrawn from the guide, which afterwards is extracted.
   The use of the device when utilizing haemostatic material is the same as before,
   The operation is over.
   Once sufficiently described the nature and one preferred embodiment of the present invention, it is only left to say that it may be possible to perform modifications on the form, the setup and the building of the assembly and its components, as well as of the application procedure, as long as said alterations do not substantially affect the characteristics of the invention which are claimed as follows:

## Claims

1. Device for guided percutaneous application of a retractile staple or a haemostatic material using a novel insertion system that opens upon withdrawal, that enables operating cardiologists and endoscopic surgeons to easily, rapidly and reliably achieve haemostasis in the puncture site, characterized in that it incorporates:
a) a perforated staple with an orifice in its center that allows to be threaded through the guide and to be guided to the puncture site.
b) an application device or applicator that holds the staple or haemostatic material, allows for the percutaneous movement of said staple to the puncture site, and its activation.

2. Device for guided percutaneous application of a retractile staple or a haemostatic material using a novel insertion system that opens upon withdrawal, according to claim 1 characterized in that a) said external unit (1) incorporates two tubular sections of different diameter (1) wherein the section with the larger diameter constitutes the housing chamber of said compression spring (3); the section of larger diameter incorporates two wings (5) designed for being handled by the fingers; said section of smaller diameter has in its end that is opposite to the section of larger diameter, an offset with some bumpers (4) directed to exert pressure on the retractile staple, and has nearby its central zone a slot (7|) to allow the exit of the guide used in the intervention: b) said inner unit (2) is built of a cylindrical shaft that shows in one of its ends a knob directed to be moved by means of a simple push with the thumb, in its central zone there is a circular protuberance that serves as bumper for the compression spring (3) and in its opposite or forwarding end, shows a spindle-shaped offset with some diminute hooks (6) for fixation of the retractile staple (8); from this spindle-shaped offset there starts a circular, preferably coaxial channel (7), that comes into an external slot provided for the exit of the guide that is used in previously performed intervention.

3. Device for guided percutaneous application of a retractile staple or a haemostatic material using a novel insertion system that opens upon withdrawal, according to claim 1 characterized in that said applicator has in its distal end an undercutting, a tunnel, and a slot that facilitates its threading through the guide and be percutaneously directed to the puncture site.

4. Device for guided percutaneous application of a retractile staple or a haemostatic material using a novel insertion system that opens upon withdrawal, according to claim 1 characterized in that said retractile staple shows a rectangular shape with a central orifice directed to permit the pass through of the guide, and for tips (9) of 0,4 and 0,5 mms that will penetrate into the tissue to be sutured, this retractile staple being provided of a notch along its opposite ridges for its engaging to the forwarding end of the inner unit (2) of the apparatus, said retractile staple being provided of a weakened central zone foreseen for being folded on the tissue as there is applied pressure on the laterals.

5. Device for guided percutaneous application of a retractile staple or a haemostatic material using a novel insertion system that opens upon withdrawal, according to claim 1 characterized in that said applicator has a pivoting system (4) that causes a retractile movement of the staple, while at the same time closing the puncture site.

6. Method for allocating retractile staples (8) or hameostatic materials by means of the application of the device according to claims 1-5 characterized in that it consists of the following phases:
a) in the initial or rest position, the retractile staple (8) appears attached through the notch (10) to the hooks (6') of the forwarding end of the inner unit (2) of the apparatus;
b) In the first action phase of the apparatus, the retractile staple (8) has been moved until contacting the bumpers (4), being it the moment in which the tips (9) of said staple penetrate the orifice to be closed (not shown in the fig).
c) In the second action phase of the apparatus, said staple (8) has been folded and moved back due to the forwarding motion of the inner unit (2) in relation to the external unit (1), because of pressing the forwarding end of he inner unit (2) on the staple against the bumpers (4), whose angles act as |fulcrum|, causing this way the closure of said staple (8) on the tissue to be sutured and in the same time its retraction or backward movement, and due to the fact that the weakened central zone of said staple induces said folding and retraction,
d) In the final phase of the intervention, the staple (8) remains attached and closed onthe tissue to be sutured, remaining released from the apparatus but still threaded to the guide, which is in the following manually extracted, the apparatus finally being withdrawn.

7. Device for guided percutaneous application of a retractile staple or a haemostatic material using a novel insertion system that opens upon withdrawal and method for allocating retractile staples or hameostatic materials according to claims 1-6 characterized in that because of employing said elements, a quick and safe haemostatic or mechanical method is achieved for acting, without having to wait for the blood clot to form.

8. Device for guided percutaneous application of a retractile staple or a haemostatic material using a novel insertion system that opens upon withdrawal, according to claim 1 characterized in that it incorporates a spring (3) for moving back to its original rest position as soon as the staple has been applied, and to facilitate the withdrawal of the device.

9. Device for guided percutaneous application of a retractile staple or a haemostatic material using a novel insertion system that opens upon withdrawal and method for allocating retractile staples or hameostatic materials according to claims 1-6 characterized in that it facilitates the introduction of said device up to the orifice of the artery.

10. Device for guided percutaneous application of a retractile staple or a haemostatic material using a novel insertion system that opens upon withdrawal, according to claim 1 characterized in that it incorporates a blocking system (13) that avoids involuntary activation of the retractile staple, before the introducing device has been withdraw
